# EUROPEAN PATENT SPECIFICATION

(11) **EP 0 799 064 B1**
(45) Date of publication and mention of the grant of the patent: **25.08.1999**
(21) Application number: 95941237.0
(22) Date of filing: 21.12.1995
(51) Int. Cl.: A61M 5/30, A61M 25/00

(54) **PARTICLE DELIVERY**
TEILCHENABGABE
ADMINISTRATION DE PARTICULES

(30) Priority: 23.12.1994 GB 9426379
(43) Date of publication of application: 08.10.1997
(73) Proprietor: PowderJect Research Limited, Oxford OX4 4GA (GB)
(72) Inventor: BELLHOUSE, Brian, John, Islip, Oxfordshire OX5 2SQ (GB); BELL, John, Islip OX5 5SG (GB)
(74) Representative: Price, Nigel John King
(86) International application number: PCT/GB95/03016
(87) International publication number: WO 96/20022

(56) References cited:
- EP-A- 0 370 571
- EP-A- 0 469 814
- WO-A-94/24263
- GB-A- 2 206 794
- US-A- 3 308 818
- US-A- 4 945 050
- US-A- 5 282 785

## Description

In our earlier WO94/24263, we disclose a non-invasive drug delivery system involving the use of a needleless syringe which fires particles of a therapeutic agent in controlled doses into body tissue, e.g. through the intact skin, or delivers genetic material into living cells. The syringe described in the earlier application is constructed as an elongate tubular nozzle, a rupturable membrane initially closing the passage through the nozzle adjacent to the upstream end of the nozzle, particles of a therapeutic agent located adjacent to the membrane, and energising means for applying to the upstream side of the membrane a gaseous pressure sufficient to burst the membrane and produce through the nozzle a supersonic gas flow in which the particles are entrained.

As explained in the earlier specification, the particles of the therapeutic agent may be carrier particles coated, for example, with genetic material, or may be powdered drugs for all kinds of therapeutic use. Similarly the earlier specification explains the parameters of particle size (preferably 10-40 µm) density (preferably 0.5-2.0 g/cm³), and velocity (preferably 200-2500 m/sec), and momentum density (preferably 4-7 kg/sec/m), which have been found to be appropriate for adequate target penetration. These parameters are unchanged but we have now devised a modification of the particle delivery system.

According to the present invention, a needleless syringe comprising a body containing a lumen, an upstream end of which is, or is arranged to be, connected to a source of gaseous pressure which can suddenly be released into the lumen to cause particles of a therapeutic agent to be delivered from the downstream end of the lumen (as disclosed in WO 94/24263) is characterised in that the downstream end of the lumen terminates behind a bistable diaphragm which is movable between an inverted position in which it presents outwardly of the body a concavity for containing the particles of a therapeutic agent, and an everted, outwardly convex, position; the arrangement being such that, in use, when the gas under pressure is released into the lumen, the diaphragm will snap over from its inverted to its everted position and catapult the particles outwardly.

This new construction has an advantage that even when, as will be usual, a gaseous supersonic shockwave will be necessary to snap the diaphragm over from its inverted to its everted position, the diaphragm will contain the gas within the lumen so that no provision has to be made for dissipating and silencing any shockwave reflected from the target. Also the target tissue is not subjected to any possibility of harm from the high speed gas flow.

As disclosed in the earlier specification, the release of the pressurized gas may be achieved by building up pressure behind a rupturable membrane until the pressure difference across the membrane is sufficient to rupture the membrane and release the gas suddenly into the lumen. Alternatively the syringe may incorporate a reservoir of compressed gas having a valve which can be opened suddenly to release the gas into the lumen. In both cases, the velocity of the shockwave is increased if the gas is lighter than air, e.g. helium. This effect is enhanced if the lumen is also initially filled with a gas, which is lighter than air, e.g. helium.

In order to avoid loss of particles prior to delivery and to maintain sterility of the particles, the concavity is preferably covered by, for example, a retractable shield or a thin barrier film which is readily penetrated by the particles upon ejection.

The syringe may be constructed for the transdermal delivery of drugs into the body, in which case the lumen may be provided by the passageway through a tubular nozzle, the diaphragm being provided adjacent to the downstream end of the nozzle and facing substantially in the axial direction of the nozzle. Alternatively, the invention may be used in conjunction with a catheter, such as an arterial catheter, in which case the diaphragm may be provided in a sidewall of the catheter body so that upon eversion, the particles are propelled laterally from the body. This would find use in vascular proliferative diseases for delivering genetic material into the wall of the expanded stenotic blood vessel, to transform genetically the endothelial cells lining the wall of the blood vessel with the aim of preventing subsequent restenosis/re-occlusion of the blood vessel.

Moreover, the development of a catheter-based delivery system may find other uses, as for localised delivery of a combination of compounds (eg for chemotherapy) into specific internal organs and for the local organ-based hormone replacement. The catheter device would also be useful in the administration of drugs or DNA to accessible surfaces for medical purposes (eg for the treatment of tumours of mucosal surfaces, such as respiratory, gastrointestinal or genito-urinary tracts).

The invention is illustrated by way of example in the accompanying drawings.

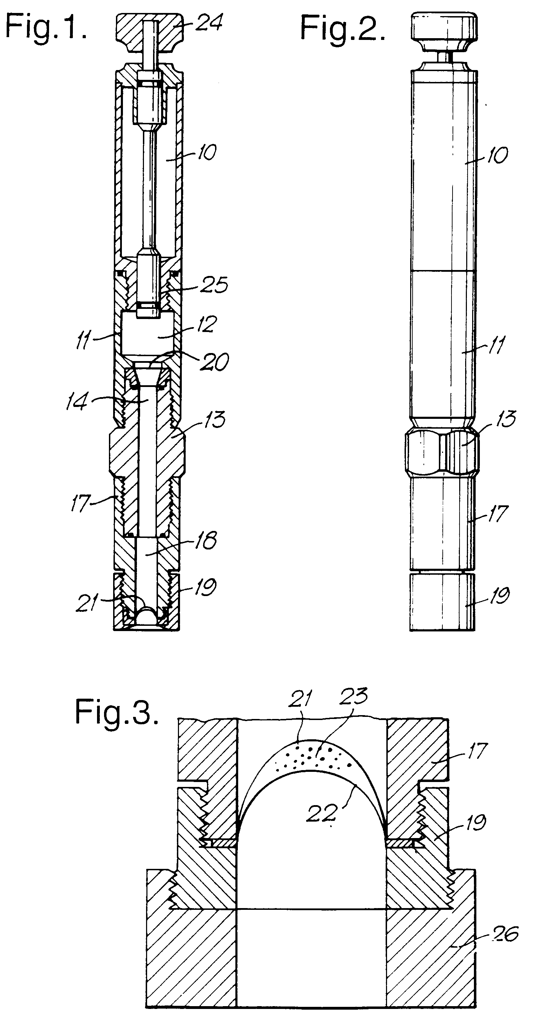

Figures 1 to 3 show a syringe for transdermal delivery of particles of therapeutic agent.

Thus as particularly shown in Figure 1 the syringe has a cylindrical reservoir 10 initially containing helium under a pressure of about 80 bar. This is screwed and sealed to a first tubular body portion 11 containing a rupture chamber 12. The body portion 11 is screwed and sealed to a second tubular body portion 13 containing a passageway 14. In turn the body portion 13 is screwed and sealed to a third tubular body portion 17 containing a passageway 18, and a tubular tip portion 19 is screwed to the bottom of the body portion 17.

With this construction the reservoir 10 can be stored separately and fitted to the rest of the syringe immediately prior to use. The body portions 11 and 13 are separable to allow the sandwiching between them of a rupturable membrane 20. The tip portion 19 is separable from the body portion 17 to allow the sandwiching between them of an invertible bistable diaphragm 21 which is shaped in the form of a dome from a stiff and strong, but resilient, material such as Mylar by thermoforming in a suitable jig. The body portions 13 and 17 are separable so that the parts 17, 19 and 21 can be provided as a disposable unit.

Particles of therapeutic agent will initially be provided in the concavity at the externally facing surface of the diaphragm 21. The particles may be attached by electrostatic forces, by their natural sticky nature, or by the evaporation of ethanol in which the particles have been suspended. Preferably, however, and for reasons of sterility, the diaphragm 21 is covered and sealed at its edge to a weak barrier film 22 to form a sealed capsule containing the particles 23, as shown in Figure 3. The weak barrier film 22 may be cut or scored to assist rupture and reduce membrane fragmentation.

In use with the syringe assembled and with the lumen 14, 18 prefilled with helium at approximately atmospheric pressure, the tip is placed in proximity to, or in contact with, the skin to be treated and a plunger 24 is depressed to open a valve 25 and allow the helium to be discharged into the rupture chamber 12. This valve 25 may preferably be arranged such that the frontal area of the plunger is greater at the downstream exit to the cylindrical reservoir 10 when compared with the upstream frontal area of the plunger, resulting in a self-opening (and quick-opening) valve. When the pressure in the chamber 12 has reached a sufficient value of, for example, about 23 bar, the membrane 20 bursts, releasing a shockwave which propagates through a lumen, i.e. a nozzle, formed by the passageways 14 and 18, and causes the diaphragm 21 suddenly to evert into a downwardly, outwardly convex, configuration. This propels the particles 23 rapidly forward out of the syringe with simultaneous rupture of the barrier film 22.

As suggested in Figure 3, a short tubular spacer 26 may be provided to reduce the velocity of the particles before impact and to enable the particles to become more spread out in order to increase the target area of skin.

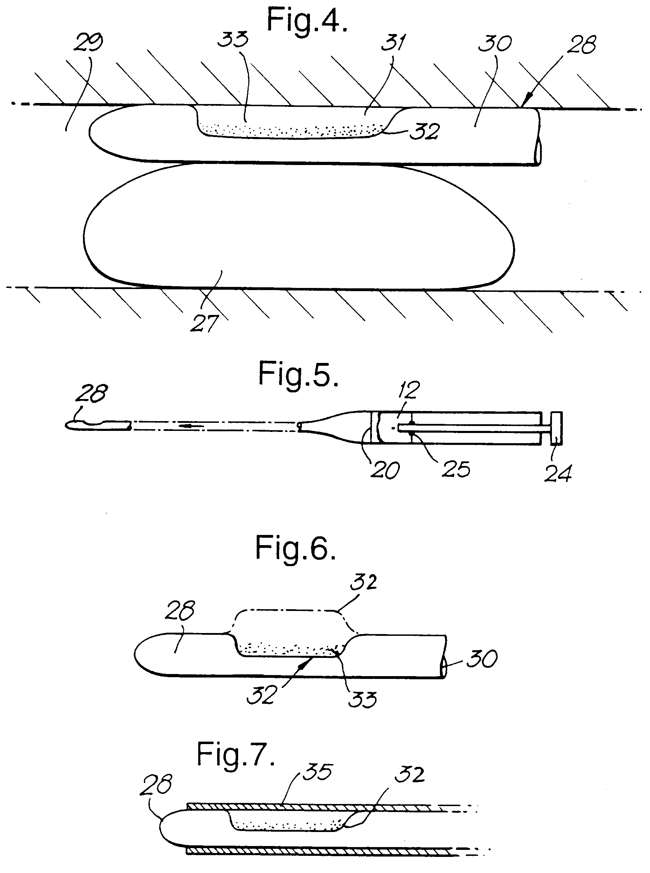

Figures 4 to 7 show the application of the invention to an arterial catheter for use in vascular proliferative disorders.

Figure 5 shows the whole catheter, provided at its upstream end with a reservoir 10, valve 25, rupture chamber 12 and rupturable membrane 20, similar to those of the first example. The catheter may be a triple lumen catheter, one lumen for the usual guidewire, the second to carry gas to inflate a positional balloon 27 for urging a body 28 adjacent to the leading end (shown in Figure 4) of the catheter against a wall of an artery 29, and the third 30 for propagating a shockwave to the tip of the catheter.

At an opening 31 in a sidewall of the catheter body 28, there is provided a bistable diaphragm 32. In a position shown in Figure 4 and in full lines in Figure 6, the diaphragm presents a laterally outwardly facing concavity in which particles of therapeutic agent, such as particles 33, e.g. containing or consisting of DNA, are located. In order to avoid the particles being washed out of the concavity by means of the blood flow in the artery the cavity may initially be covered by a barrier film or by a retractable sleeve 35 as shown in Figure 7.

The catheter is used analogously to the syringe of Figures 1 to 3. Thus release of helium from the reservoir 10 into the rupture chamber 12 eventually bursts the membrane 20 and causes a shockwave to be propagated along the lumen 30 causing the diaphragm 32 to evert suddenly to the dotted line position shown in Figure 6 and hence to propel the particles 33 outwardly, after retraction of the sleeve 35 or through the barrier film, into the wall of the artery 29.

Contrary to what is shown in Figure 6, it may be desirable for the diaphragm 32 to be within the peripheral envelope of the catheter body, not only when in its inverted position in order to avoid interference during insertion of the catheter, but also when in its everted position to avoid potentially damaging impact with the arterial wall.

## Claims

1. A needleless syringe comprising a body (13,17;28) containing a lumen (14,18;30), an upstream end of which is, or is arranged to be, connected to a source (10) of gaseous pressure which can suddenly be released into the lumen to cause particles of a therapeutic agent to be delivered from the downstream end of the lumen; characterised in that the downstream end of the lumen terminates behind a bistable diaphragm (21,32) which is movable between an inverted position in which it presents outwardly of the body a concavity for containing the particles (23,33) of a therapeutic agent, and an everted, outwardly convex, position; the arrangement being such that, in use, when the gas under pressure is released into the lumen, the diaphragm will snap over from its inverted to its everted position and catapult the particles outwardly.

2. A syringe according to claim 1, wherein the upstream end of the lumen (14,18;30) is initially closed by a rupturable membrane (20) which, when subjected to gas under sufficient pressure is arranged to rupture and release the gas suddenly into the lumen.

3. A syringe according to claim 1 or claim 2, in which the lumen (14,18;30) initially contains a gas which is lighter than air.

4. A syringe according to any one of the preceding claims, in which particles (23,33) of a therapeutic agent are located in the concavity of the inverted diaphragm.

5. A syringe according to claim 4, in which the particles in the concavity are covered by a retractable shield (35).

6. A syringe according to claim 4, in which the particles in the cavity are covered by a thin barrier film (22) which is readily penetrated by the particles upon ejection.

7. A syringe according to any one of the preceding claims, in which the lumen-containing body is a tubular nozzle (13,17), the diaphragm (21) being provided adjacent to the downstream end of the nozzle and facing substantially in the axial direction of the nozzle.

8. A syringe according to claim 7, in which a tubular spacer (26) projects from the nozzle downstream of the diaphragm.

9. A syringe according to any one of claims 1 to 6, in which the lumen-containing body is a catheter (28).

10. A syringe according to claim 9, in which the diaphragm (32) is provided in a side wall of the catheter body so that, upon eversion of the diaphragm, the particles (33) are propelled laterally from the body.

## Patentansprüche

1. Nadellose Spritze, umfassend einen Körper (13, 17; 28), der ein Lumen (14, 18; 30) enthält, dessen stromaufwärts gelegenes Ende mit einer Quelle (10) für Gasdruck verbunden oder zum Verbinden mit einer solchen ausgelegt ist, welcher plötzlich in das Lumen freigegeben werden kann, so daß das Austragen von Partikeln eines therapeutischen Mittels von dem stromabwärts gelegenen Ende des Lumens veranlaßt wird; dadurch gekennzeichnet, daß das stromabwärts gelegene Ende des Lumens hinter einer bistabilen Membran (21, 32) endet, die zwischen einer nach innen gekehrten Position, in der sie außerhalb des Körpers einen konkaven Raum bildet, der die Partikel (23, 33) eines therapeutischen Mittels enthält, und einer nach außen gekehrten, nach außen konvexen Position beweglich ist; wobei die Anordnung so ist, daß bei der Verwendung, wenn das unter Druck stehende Gas in das Lumen freigegeben wird, die Membran aus ihrer nach innen gekehrten in ihre nach außen gekehrte Position umschnappt und die Partikel nach außen katapultiert.

2. Spritze nach Anspruch 1, bei welcher das stromaufwärts gelegene Ende des Lumens (14, 18; 30) anfänglich durch eine zerreißbare Membran (20) verschlossen ist, die so ausgelegt ist, daß sie dann, wenn sie Gas unter ausreichendem Druck ausgesetzt ist, zerreißt und das Gas plötzlich in das Lumen freigibt.

3. Spritze nach Anspruch 1 oder Anspruch 2, bei welcher das Lumen (14, 18; 30) anfänglich ein Gas enthält, das leichter als Luft ist.

4. Spritze nach einem der vorstehenden Ansprüche, bei welcher Partikel (23, 33) eines therapeutischen Mittels in dem konkaven Hohlraum der nach innen gekehrten Membran angeordnet sind.

5. Spritze nach Anspruch 4, bei welcher die Partikel in dem konkaven Hohlraum von einer zurückziehbaren Abdeckung (35) bedeckt sind.

6. Spritze nach Anspruch 4, bei welcher die Partikel in dem konkaven Hohlraum durch eine dünne Folienbarriere (22) bedeckt sind, die beim Ausstoßvorgang ohne weiteres von den Partikeln durchdrungen wird.

7. Spritze nach einem der vorstehenden Ansprüche, bei welcher der das Lumen enthaltende Körper eine rohrförmige Düse (13,17), ist, wobei die Membran (21) dem stromabwärts gelegenen Ende der Düse benachbart vorgesehen ist und im wesentlichen in axialer Richtung der Düse weist.

8. Spritze nach Anspruch 7, bei welcher ein rohrförmiger Abstandhalter (26) von der Düse stromabwärts der Membran vorragt.

9. Spritze nach einem der Ansprüche 1 bis 6, bei welcher der das Lumen enthaltende Körper ein Katheter (28) ist.

10. Spritze nach Anspruch 9, bei welcher die Membran (32) in einer Seitenwand des Katheterkörpers vorgesehen ist, so daß dann, wenn die Membran nach außen umschlägt, die Partikel (33) von dem Körper seitlich ausgestoßen werden.

## Revendications

1. Seringue sans aiguille comprenant un corps (13, 17 ; 28), contenant une lumière (14, 18 ; 30) dont une extrémité amont est, ou est agencée, pour être, reliée à une source (10) de pression gazeuse qui peut subitement être libérée dans la lumière pour amener des particules d'un agent thérapeutique à être délivrées depuis l'extrémité aval de la lumière, caractérisée en ce que l'extrémité aval de la lumière se termine derrière un diaphragme bistable (21, 32) qui est déplaçable entre une position rentrée dans laquelle il présente à l'extérieur du corps une concavité pour contenir les particules (23, 33) d'un agent thérapeutique, et une position retournée, convexe vers l'extérieur ; l'agencement étant tel que, pendant l'utilisation, quand le gaz sous pression est libéré dans la lumière, le diaphragme passe rapidement de sa position rentrée à sa position retournée et catapulte les particules vers l'extérieur.

2. Seringue selon la revendication 1, dans laquelle l'extrémité amont de la lumière (14, 18 ; 30) est initialement fermée par une membrane (20) pouvant se rompre qui, quand elle est soumise à un gaz sous une pression suffisante, est agencée pour se rompre et libérer subitement le gaz dans la lumière.

3. Seringue selon la revendication 1 ou la revendication 2, dans laquelle la lumière (14, 18 ; 30) contient initialement un gaz qui est plus léger que l'air.

4. Seringue selon l'une quelconque des revendications précédentes, dans laquelle des particules (23, 33) d'un agent thérapeutique sont situées dans la concavité du diaphragme rentré.

5. Seringue selon la revendication 4, dans laquelle les particules dans la concavité sont recouvertes par un écran rétractable (35).

6. Seringue selon la revendication 4, dans laquelle les particules dans la cavité sont recouvertes par un mince film formant barrière (22) qui est aisément pénétré par les particules lors de l'éjection.

7. Seringue selon l'une quelconque des revendications précédentes, dans laquelle le corps contenant une lumière est une buse tubulaire (13, 17), le diaphragme (21) étant disposé en position adjacente à l'extrémité aval de la buse et étant tourné sensiblement dans la direction axiale de la buse.

8. Seringue selon la revendication 7, dans laquelle un élément d'écartement tubulaire (26) fait saillie de la buse en aval du diaphragme.

9. Seringue selon l'une quelconque des revendications 1 à 6, dans laquelle le corps contenant une lumière est un cathéter (28).

10. Seringue selon la revendication 9, dans laquelle le diaphragme (32) est disposé dans une paroi latérale du corps du cathéter de sorte que, lors du retournement du diaphragme, les particules (33) sont propulsées latéralement par rapport au corps.
